# EUROPEAN PATENT SPECIFICATION

(11) **EP 3 561 462 B1**
(45) Date of publication and mention of the grant of the patent: **27.04.2022**
(21) Application number: 19150294.7
(22) Date of filing: 04.01.2019
(51) Int. Cl.: G01G 19/50, A61B 5/00

(54) **BODY ANALYSER**
KÖRPERANALYSATOR
ANALYSEUR DE CORPS

(30) Priority: 27.04.2018 CN 201810389943; 27.04.2018 CN 201820624844 U; 27.07.2018 CN 201810847162; 27.07.2018 CN 201821214351 U
(43) Date of publication of application: 30.10.2019
(73) Proprietor: Xu, Xinqiang, Shenzhen City, Guangdong 518051 (CN)
(72) Inventor: Xu, Xinqiang, Shenzhen City, Guangdong 518051 (CN)
(74) Representative: Cabinet Laurent & Charras

(56) References cited:
- EP-A1- 1 514 513
- CN-U- 205 317 332
- CN-U- 205 597 892
- US-A- 6 038 465

## Description

### TECHNICAL FIELD

The present disclosure relates to household health care technologies, and more particularly relates to a body analyzer.

### BACKGROUND

With an improvement of people's living standards, a demand for health increases accordingly and body analyzer becomes a popular product. The body analyzer is a health care device that can measure weight, body fat, body water, body muscle, basal metabolism, body mass index, bone mass, and other compositions.

Currently, the conventional body analyzer cannot measure a height of a subject. Other height measuring instrument is required to measure the height of the subject, which brings inconvenience to the use thereof.

The documents CN205317332 and US6038465 disclose body analyzers according to the state of the art.

### SUMMARY

According to various embodiments of the present disclosure, a body analyzer is provided.

A body analyzer according to the invention is disclosed in claim 1.

The body analyzer further includes an acoustic indicator device electrically connected to the signal processor, which is used to broadcast processed data transmitted from the signal processor in voice.

The main body further includes a display screen connected to the signal processor and used to receive and display processed data transmitted from the signal processor.

The wireless communication device is at least one selected from the group consisting of a Wi-Fi device, a Bluetooth device, a ZigBee device, a Zwave device, a WLAN device, a GSM device, a 2G device, a 3G device, a 4G device, and a 5G devices.

The body analyzer further includes a power supply device used to supply power to the body analyzer.

The body composition is at least one selected from the group consisting of weight, body fat, body water, body muscle, basal metabolism, body mass index, bone mass, and other compositions.

The detecting board is a plastic plate or a cell phone.

The aforementioned body analyzer includes the main body, the signal emitter, and the signal receiver. The signal emitter and the signal receiver cooperatively detect the height data, that is, a measured distance between the platform of the main body and the detecting board is actually the height of the subject. The wireless communication device is used to perform data interaction with the external mobile device or server via network. The information transmitter is used to transmit the data of the signal emitter and the signal receiver to the weighing and body analyzing mechanism. The signal processor processes the data of the signal emitter, the signal receiver, the weighing and body analyzing mechanism, and transmits the processed data to the display screen for display and transmits the processed data to the external mobile device or server via the wireless communication device. In this way, the accurate weight and height data of the subject can be obtain, which can avoid manually height measurement via mechanical means, and ensures an accuracy of a measuring result. The body analyzer is light in size, it is easy to be collected or can move at will. The body analyzer is wirelessly connected with the network so as to transmit the data to the cloud server. The analyzed data is pushed to the mobile terminal designated by the subject, so that the subject can view changes in the body's various indicators at any time to effectively control their physical fitness.

### BRIEF DESCRIPTION OF THE DRAWINGS

FIG. 1a is a top view of a main body of a body analyzer in accordance with a first embodiment.
FIG. 1b is a side view of the body analyzer in accordance with the first embodiment in a using status.
FIG. 2 is a block diagram of a body analyzer of FIG. 1b.
FIG. 3a is a top view of a main body of a body analyzer in accordance with an example that does not correspond to the invention.
FIG. 3b is a side view of the body analyzer in accordance with the example of Fig 3b that does not correspond to the invention.
FIG. 4 is a block diagram of a body analyzer of FIG. 3b.

### DETAILED DESCRIPTION OF THE EMBODIMENTS

Reference will now be made to the drawings to describe, in detail, embodiments of the present camera module. It should be noted that references to "an" or "one" embodiment in this disclosure are not necessarily to the same embodiment, and such references mean at least one.

Unless the context clearly requires otherwise, throughout the description and the claims, the words "comprise," "comprising," and the like are to be construed in an inclusive sense as opposed to an exclusive or exhaustive sense; that is to say, in the sense of "including, but not limited to." Words using the singular or plural number also include the plural or singular number respectively. Additionally, the words "herein,""above," "below" and words of similar import, when used in this application, shall refer to this application as a whole and not to any particular portions of this application. When the claims use the word "or" in reference to a list of two or more items, that word covers all of the following interpretations of the word: any of the items in the list, all of the items in the list and any combination of the items in the list.

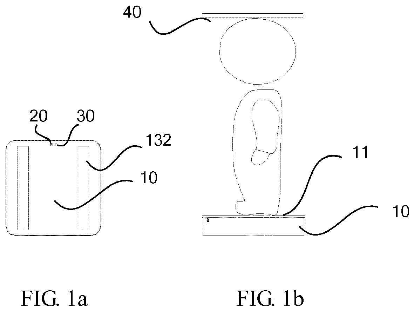

Referring to FIGS. 1a and 1b, a body analyzer 100 according to a first embodiment is provided. In this embodiment, the body analyzer 100 includes a main body 10 and a detecting board 40.

The main body 10 includes a platform 11, which can be in contact with feet of a subject, such as an adult, or a child. In one embodiment, the main body 10 is provided with a signal emitter 20 and a signal receiver 30. The signal emitter 20 is used to emit a signal for measuring a height of the subject. The signal emitter 20 can be at least one selected from a group consisting of a laser emitter, an LED emitter, an infrared emitter. The signal receiver 30 is used to receive the height measuring signal reflected by the detecting board 40.

The detecting board 40 is separated from the main body 10 and is used to abut against a top head of the subject. The detecting board 40 can be moved towards or away from the platform 11. When abutting against the top head of the subject, the detecting board 40 can reflect the signal emitted from the signal emitter 20 to the signal receiver 30. The detecting board 40 can be any planar board that can achieve a signal reflection, such as a plastic plate or even a cell phone.

When measuring, the subject steps on the platform 11 and holds the detecting board 40 on the top head thereof. The signal emitter 20 emits the height measuring signal towards the detecting board 40. The height measuring signal is reflected by the detecting board 40 and is received by the signal receiver 30, thereby achieving the height measuring of the subject.

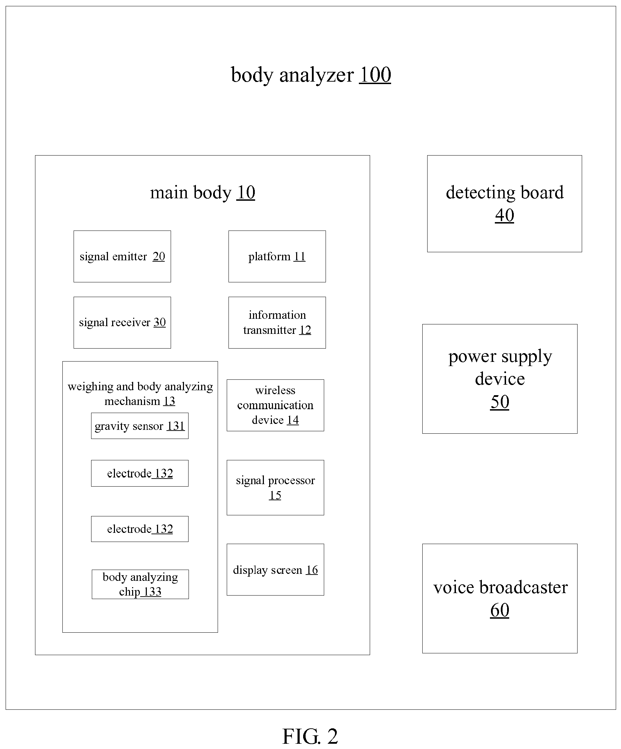

Referring to FIG. 2, the main body 10 further includes an information transmitter 12, a weighing and body analyzing mechanism 13, a wireless communication device 14, a signal processor 15, and a display screen 16.

The information transmitter 12 is electrically connected to the signal emitter 20, the signal receiver 30, and the weighing and body analyzing mechanism 13, respectively. The information transmitter 12 is used to transmit data of the signal emitter 20 and the signal receiver 30 to the weighing and body analyzing mechanism 13.

The weighing and body analyzing mechanism 13 receives the data transmitted by the information transmitter 12 and calculates body compositions. It should be understood that the data transmitted by the information transmitter 12 and received by the weighing and body analyzing mechanism 13 can be used in the calculation or determination of the body compositions, or the body compositions can be calculated or determined without the data transmitted by the information transmitter 12 and received by the weighing and body analyzing mechanism 13. In one embodiment, the weighing and body analyzing mechanism 13 can calculate or determine body compositions based on bioelectrical impedance analysis (BIA) technology. The body compositions can be at least one selected from a group consisting of weight, body fat, body water, body muscle, basal metabolism, body mass index, bone mass, and other compositions. In one embodiment, the weighing and body analyzing mechanism 13 includes a gravity sensor 131, two electrodes 132, and a body analyzing chip 133. The detail measurement processes of the body compositions such as body fat, body water, body muscle, basal metabolism, body mass index, and bone mass can be found in Chinese Patent Applications NO. CN205317331U, and CN204909395U, the disclosure content of which are incorporated herein by reference in its entirety. It should be understood that in other embodiments, the number of the electrodes can be 4, 6, and other quantities.

Body Analyzer that utilizes a "foot-to-foot" BIA(bioelectrical impedance analysis ) technology to determine internal body composition.

which is used to measure a weight and body compositions of the subject.

The wireless communication device 14 is used to interact with an external mobile device or server via a wireless network. The wireless communication device can be at least one selected from a group consisting of a Wi-Fi device, a Bluetooth device, a ZigBee device, a Zwave device, a WLAN device, a GSM device, a 2G device, a 3G device, a 4G device, and a 5G devices.

The signal processor 15 is electrically connected to the signal emitter 20, the signal receiver 30, the weighing and body analyzing mechanism 13, the wireless communication device 14, and the display screen 16, respectively. The signal processor 15 can be a microprocessor, or other chips that can implement data processing, etc. The signal processor 15 processes data transmitted from the signal emitter 20 and the signal receiver 30 to obtain height data of the subject, processes data transmitted by the weighing and body analyzing mechanism 13 to obtain weight and body compositions data of the subject, and transmits the height data and the weight and body compositions data to the display screen 16. The signal processor 15 further transmits the height data and the weight and body compositions data to the external mobile device or server via the wireless communication device 14. The external mobile device or server can store the height data and the weight and body compositions data, or display a constitutional index of the subject via a certain manner (such as curve diagram, etc.), thereby establishing a relatively comprehensive human health index of the subject and making it convenient for a user to have an intuitive understanding of the constitutional index of the subject.

The display screen 16 receives and displays the height data and the weight and body compositions data transmitted by the signal processor 15. The display screen 15 can be an LCD or a digital display screen.

In this embodiment, the body analyzer 100 further includes a power supply device 50. The power supply device 50 is configured to supply power to the body analyzer 100. The power supply device 50 can be a battery or other rechargeable device. In one embodiment, the power supply device can convert an external alternating current into a direct current to supply power to the body analyzer 100.

In this embodiment, the body analyzer 100 further includes an acoustic indicator device 60 electrically connected to the signal processor 15. The acoustic indicator device 60 is used to broadcast measuring results transmitted by the signal processor 15 in voice.

The aforementioned body analyzer 100 includes the main body 10 and the detecting board 40 separated from the main body 10. The main body 10 includes the signal emitter 20, the signal receiver 30, the information transmitter 12, the weighing and body analyzing mechanism 13, the wireless communication device 14, the signal processor 15, and the display screen 16. The signal emitter 20 emits a height measuring signal. The height measuring signal is received by the signal receiver 30 after being reflected by the detecting board 40. A measured distance between the platform 11 and the detecting board 40 is actually the height of the subject. The wireless communication device 14 interacts with the external mobile device or server via the wireless network. The signal processor 15 is electrically connected to the signal emitter 20, the signal receiver 30, the information transmitter 12, the weighing and body analyzing mechanisml3, the wireless communication device 14 and the display screen 16, respectively. The signal processor 15 processes the data of the signal emitter 20, the signal receiver 30, the weighing and body analyzing mechanism 13, and transmits the processed data to the display screen 16 for display and transmits the processed data to the external mobile device or server via the wireless communication device 14. In this way, the accurate weight and height data of the subject can be obtain via the processing of the signal processor 15 on the received signal, which avoids manually height measurement via mechanical means, and ensures an accuracy of a measuring result. The measuring result can be visually displayed on the display screen and can be transmitted to the external mobile device or server for storage via the wireless communication device, thereby avoiding manually record of the measuring result, which saves time and labor, and is convenient and practical.

In an alternative embodiment, the main body 10 can be further provided with an operation button (not shown). The operation button can be at least one selected from a group consisting of a lock button, an acoustic indicating button, a wireless transmission button, a power button, and a height display button. The lock button is used to prevent display values of the weight and body compositions data from frequently changing due to shaking of the subject on the body analyzer 100. When the subject presses the lock button, the display values of the weight and body compositions data thereof are no longer changed. The acoustic indicating button is used to provide a choice for the subject about whether broadcasting the measuring results (the height, weight, and body compositions data, etc.) thereof in voice or not. When the acoustic indicating button is pressed, the body analyzer 100 broadcasts the measuring results, otherwise the broadcasting is not performed. The wireless transmission button is used to provide a choice for the subject about whether transmitting the measuring results to the external mobile device or server via the wireless network. When the wireless transmission button is pressed, the body analyzer 100 transmits the measuring results to the external mobile device or server, otherwise the transmission of the measuring results is not performed. The power button is used to control whether energizing the body analyzer 100 or not. When the power button is pressed, the body analyzer 100 is powered on, otherwise the body analyzer 100 is not powered. The height display button is used to provide a choice for the subject about whether measuring and displaying the height data thereof. A default state of the height display button indicates to measure and display the height data. When the height display button is pressed, the height measurement and display are not performed.

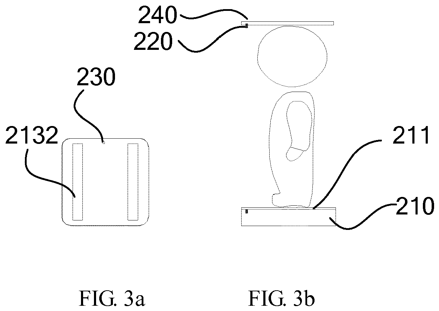

Referring to FIGS. 3a to 3b, a body analyzer 200 according to an example that does not correspond to the invention is provided. The body analyzer 200 is similar to the body analyzer 100.

Correspondingly, in this example that does not correspond to the invention, the body analyzer 200 includes a main body 210 and a detecting board 240. The main body 210 includes a platform 211, which can be in contact with feet of a subject. The detecting board 40 is separated from the main body 10, and is used to abut against a top head of the subject. A difference between the body analyzer 200 of this example and the body analyzer 100 of the first embodiment is that the detecting board 240 is provided with a signal emitter 220, and the main body 210 is provided with a signal receiver 230. The signal emitter 220 is used to emit a signal for measuring a height of the subject. The signal receiver 230 is used to receive the height measuring signal transmitted by the signal emitter 220. When measuring, the subject steps on the platform 211 and holds the detecting board 240 abutting against the top head thereof, so that the signal transmitted by the signal emitter 220 can be received by the signal receiver 230.

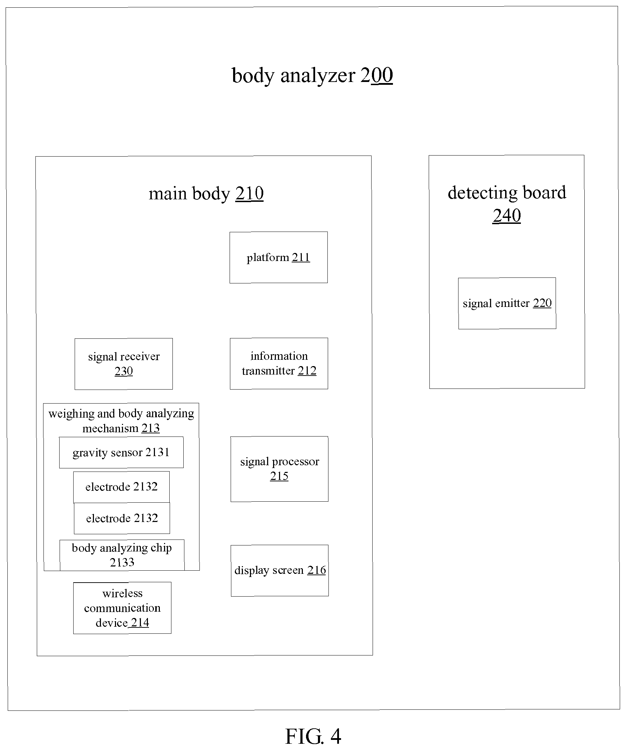

Referring to FIG. 4, the body analyzer 210 further includes an information transmitter 212, a weighing and bodying analyzing mechanism 213, a wireless communication device 214, a signal processor 215, and a display screen 216.

The information transmitter 212 is electrically connected to the signal receiver 230, and the weighing and body analyzing mechanism 213, respectively. The information transmitter 212 is used to transmit data received by the signal receiver 230 to the weighing and body analyzing mechanism 213.

The weighing and body analyzing mechanism 213 receives the data transmitted by the information transmitter 212. The weighing and body analyzing mechanism 213 is used to calculate body compositions. In one embodiment, the weighing and body analyzing mechanism 213 can calculate or determine body compositions based on bioelectrical impedance analysis (BIA) technology. The body compositions can be at least one selected from a group consisting of weight, body fat, body water, body muscle, basal metabolism, body mass index, bone mass, and other compositions. In one embodiment, the weighing and body analyzing mechanism 213 includes a gravity sensor 2131, two electrodes 2132, and a body analyzing chip 2133. The detail measurement processes of the body compositions such as body fat, body water, body muscle, basal metabolism, body mass index, and bone mass can be found in Chinese Patent Applications NO. CN205317331U, and CN204909395U, the disclosure content of which are incorporated herein by reference in its entirety.

The wireless communication device 214 is used to interact with an external mobile device or server via a wireless network.

The signal processor 215 is electrically connected to the signal receiver 230, the weighing and body analyzing mechanism 213, the wireless communication device 214, and the display screen 216, respectively. The signal processor 215 processes data transmitted of the signal emitter 220 and the signal receiver 230 to obtain height data of the subject, processes data transmitted by the weighing and body analyzing mechanism 213 to obtain weight and body compositions data of the subject, and transmits the height data and the weight and body compositions data to the display screen 216. The display screen 216 can display the height data and the weight and body compositions data transmitted by the signal processor 215. The signal processor 215 further transmits the height data and the weight and body compositions data to the external mobile device or server for storage via the wireless communication device 214.

## Claims

1. A body analyzer (100), comprising: a main body (10) comprising a platform (11) in contact with feet of a subject; a detecting board (40) separated from the main body (10) and configured to abut against a top head of the subject, the detecting board (40) being capable of moving towards or away from the platform (11); wherein the main body (10) comprises an information transmitter (12), a weighing and body analyzing mechanism (13), a signal processor (15), and a wireless communication device (14); the information transmitter (12) is configured to transmit data of a signal emitter (20) and a signal receiver (30) to the weighing and body analyzing mechanism (13); the weighing and body analyzing mechanism (13) is configured to calculate body compositions of the subject; the signal processor (15) is configured to process data from the signal emitter (20), the signal receiver (30), and the weighing and body analyzing mechanism (13); and the wireless communication device (14) is configured to transmit a processed data to an external mobile device or server via a wireless network **characterized in that** the main body (10) is further provided with the signal emitter (20) and the signal receiver (30), the detecting board (40) is configured to reflect the signal emitted from said signal emitter (20) to said signal receiver (30), and the signal emitter (20) and the signal receiver (30) are configured to measure a distance between the platform (11) and the detecting board (40); and the signal emitter (20) is at least one selected from the group consisting of a laser emitter, an LED emitter, an infrared emitter.

2. . The body analyzer (100) according to claim 1, further comprising an acoustic indicator device (60) electrically connected to the signal processor (15), and configured to broadcast processed data transmitted from the signal processor (15) in voice.

3. . The body analyzer (100) according to claim 1, **characterized in that** the main body (10) further comprises a display screen (16) connected to the signal processor (15) and configured to receive and display processed data transmitted from the signal processor (15).

4. . . The body analyzer (100) according to claim 1, **characterized in that** the wireless communication device (14) is at least one selected from the group consisting of a Wi-Fi device, a Bluetooth device, a ZigBee device, a Zwave device, a WLAN device, a GSM device, a 2G device, a 3G device, a 4G device, and a 5G devices.

5. . The body analyzer (100) according to claim 1, further comprising a power supply device (50) configured to supply power to the body analyzer (100).

6. . The body analyzer (100) according to claim 1, **characterized in that** the body composition is at least one selected from the group consisting of weight, body fat, body water, body muscle, basal metabolism, body mass index, bone mass.

7. . The body analyzer (100) according to claim 1, **characterized in that** the detecting board (40) is a plastic plate or a cell phone.

## Patentansprüche

1. Ein Körperanalysator (100), mit einem Hauptkörper (10), umfassend eine Plattform (11), in Kontakt mit den Füßen einer Testperson, eine Erfassungstafel (40), getrennt vom Hauptkörper (10) und dazu konfiguriert, gegen den Oberkopf der Testperson zu stoßen, die Erfassungstafel (40) ist dabei in der Lage sich hin zu oder weg von der Plattform (11) zu bewegen; wobei der Hauptkörper (10) einen Informationssender (12), einen Wiege- und Körperanalysemechanismus (13), einen Signalprozessor (15), und eine drahtlose Kommunikationsvorrichtung (14) enthält; der Informationssender (12) ist dazu konfiguriert, Daten eines Signalgebers (20) und eines Signalempfänger (30) an den Wiege- und Körperanalysemechanismus (13) zu übertragen; der Wiege- und Körperanalysemechanismus (13) ist dazu konfiguriert die Körperzusammensetzung der Testperson zu berechnen; der Signalprozessor (15) ist dazu konfiguriert, Daten vom Signalgeber (20), dem Signalempfänger (30), und dem Wiege- und Körperanalysemechanismus (13) zu verarbeiten; und die drahtlose Kommunikationsvorrichtung (14) ist dazu konfiguriert, verarbeitete Daten über ein drahtloses Netzwerk an ein externes mobiles Gerät oder einen Server zu senden. **dadurch gekennzeichnet, dass** der Hauptkörper (10) außerdem mit dem Signalgeber (20) und dem Signalempfänger (30) ausgerüstet ist, die Erfassungstafel (40) ist dazu konfiguriert, das von diesem Signalgeber (20) ausgegebene Signal an diesen Signalempfänger (30) zu reflektieren, und der Signalgeber (20) und der Signalempfänger (30) sind dazu konfiguriert, den Abstand zwischen der Plattform (11) und der Erfassungstafel (40) zu messen; und beim Signalgeber (20) handelt es sich um mindestens ein Gerät, ausgewählt aus der Gruppe bestehend aus einem Laseremitter, einem LED- Emitter und einem Infrarot-Emitter.

2. Körperanalysator (100) nach Anspruch 1, weiterhin umfassend eine akustische Anzeigevorrichtung (60), elektrisch verbunden mit dem Signalprozessor (15), und dazu konfiguriert, verarbeitete Daten, übertragen vom Signalprozessor (15) in Sprachform zu senden.

3. Körperanalysator (100) nach Anspruch 1, **dadurch gekennzeichnet, dass** der Hauptkörper (10) außerdem einen Anzeigebildschirm (16) umfasst, verbunden mit dem Signalprozessor (15) und konfiguriert zum Empfang und Anzeige verarbeiteter Daten, die vom Signalprozessor (15) übertragen werden.

4. Körperanalysator (100) nach Anspruch 1, **dadurch gekennzeichnet, dass** die drahtlose Kommunikationsvorrichtung (14) mindestens eine aus der Gruppe bestehend aus einem Wi-Fi- Gerät, einem Bluetooth- Gerät, einem ZigBee- Gerät, einem Zwave- Gerät, einem WLAN- Gerät, einem GSM- Gerät, einem 2G- Gerät, einem 3G- Gerät, einem 4G- Gerät und einem 5G- Gerät ist

5. Körperanalysator (100) nach Anspruch 1, weiterhin umfassend ein Stromversorgungsgerät (50), konfiguriert um Strom an den Körperanalysator (100) zu liefern.

6. Körperanalysator (100) nach Anspruch 1, **dadurch gekennzeichnet, dass** die Körperzusammensetzung mindestens ein Wert aus der Gruppe bestehend aus Gewicht, Körperfett, Körperwasser, Körpermuskeln, basalem Metabolismus, Body Mass Index, Knochenmasse ist.

7. Körperanalysator (100) nach Anspruch 1, **dadurch gekennzeichnet, dass** die Erfassungstafel (40) eine Kunststoffplatte oder ein Handy ist.

## Revendications

1. Analyseur de corps (100), comprenant : un corps principal (10) comprenant une plateforme (11) en contact avec les pieds d'un sujet ; une plaque de détection (40) séparée du corps principal (10) et configurée pour venir en butée contre le dessus de la tête du sujet, la plaque de détection (40) étant capable de se rapprocher ou de s'éloigner de la plateforme (11) ; dans lequel le corps principal (10) comprend un transmetteur d'informations (12), un mécanisme de pesée et d'analyse corporelle (13), un processeur de signal (15) et un dispositif de communication sans fil (14) ; le transmetteur d'informations (12) est configuré pour transmettre des données d'un émetteur de signal (20) et d'un récepteur de signal (30) au mécanisme de pesée et d'analyse de corps (13) ; le mécanisme de pesée et d'analyse de corps (13) est configuré pour calculer des compositions corporelles du sujet ; le processeur de signal (15) est configuré pour traiter des données provenant de l'émetteur de signal (20), du récepteur de signal (30) et du mécanisme de pesée et d'analyse corporelle (13) ; et le dispositif de communication sans fil (14) est configuré pour transmettre des données traitées à un dispositif mobile ou serveur externe via un réseau sans fil, **caractérisé en ce que** le corps principal (10) est en outre muni de l'émetteur de signal (20) et du récepteur de signal (30), la plaque de détection (40) est configurée pour réfléchir le signal émis depuis ledit émetteur de signal (20) vers ledit récepteur de signal (30), et l'émetteur de signal (20) et le récepteur de signal (30) sont configurés pour mesurer une distance entre la plateforme (11) et la plaque de détection (40) ; et l'émetteur de signal (20) est au moins un élément sélectionné dans le groupe constitué d'un émetteur laser, d'un émetteur à DEL, d'un émetteur infrarouge.

2. Analyseur de corps (100) selon la revendication 1, comprenant en outre un dispositif indicateur acoustique (60) connecté électriquement au processeur de signal (15), et configuré pour diffuser des données traitées transmises depuis le processeur de signal (15) de manière vocale.

3. Analyseur de corps (100) selon la revendication 1, **caractérisé en ce que** le corps principal (10) comprend en outre un écran d'affichage (16) connecté au processeur de signal (15) et configuré pour recevoir et afficher des données traitées transmises à partir du processeur de signal (15).

4. Analyseur de corps (100) selon la revendication 1, **caractérisé en ce que** le dispositif de communication sans fil (14) est au moins un sélectionné dans le groupe constitué d'un dispositif Wi-Fi, d'un dispositif Bluetooth, d'un dispositif ZigBee, d'un dispositif Zwave, d'un dispositif WLAN, d'un dispositif GSM, d'un dispositif 2G, d'un dispositif 3G, d'un dispositif 4G et de dispositifs 5G.

5. Analyseur de corps (100) selon la revendication 1, comprenant en outre un dispositif d'alimentation en énergie (50) configuré pour fournir de l'énergie à l'analyseur de corps (100).

6. Analyseur de corps (100) selon la revendication 1, **caractérisé en ce que** la composition corporelle est au moins une composition choisie dans le groupe constitué par le poids, la graisse corporelle, l'eau corporelle, les muscles corporels, le métabolisme de base, l'indice de masse corporelle, la masse osseuse.

7. Analyseur de corps (100) selon la revendication 1, **caractérisé en ce que** la plaque de détection (40) est une plaque en plastique ou un téléphone cellulaire.
